# EUROPEAN PATENT SPECIFICATION

(11) **EP 0 824 517 B1**
(45) Date of publication and mention of the grant of the patent: **24.07.2002**
(21) Application number: 96916111.6
(22) Date of filing: 09.05.1996
(51) Int. Cl.: C07D 211/90

(54) **A PROCESS FOR THE PREPARATION OF LERCANIDIPINE HYDROCHLORIDE**
VERFAHREN ZUR HERSTELLUNG VON LERCANIDIPIN HYDROCHLORID
PROCEDE DE PREPARATION D'HYDROCHLORURE DE LERCANIDIPINE

(30) Priority: 12.05.1995 IT MI950957
(43) Date of publication of application: 25.02.1998
(73) Proprietor: RECORDATI S.A. CHEMICAL and PHARMACEUTICAL COMPANY, 6830 Chiasso (CH); RECORDATI INDUSTRIA CHIMICA E FARMACEUTICA S.p.a., 20148 Milano (IT)
(72) Inventor: LEONARDI, Amedeo, I-20154 Milano (IT); MOTTA, Gianni, I-20030 Barlassina (IT)
(74) Representative: SERJEANTS
(86) International application number: EP9602122
(87) International publication number: WO9635668

(56) References cited:
- EP-A- 0 153 016
- DRUGS OF THE FUTURE, vol. 20, no. 12, 1995, pages 1284-1285, XP002010272 RECORDATI ET AL.: "Lercanidipine hydrochloride"
- DRUGS OF THE FUTURE, vol. 12,no. 12,1987, pages 1113 -1115, RECORDATI

## Description

The invention relates to a process for the preparation of lercanidipine hydrochloride.

Lercanidipine is methyl 1,1,N-trimethyl-N-(3,3-diphenylpropyl)-2-aminoethyl 1,4-dihydro-2,6-dimethyl-4-(3-nitrophenyl)-pyridine-3,5-dicarboxylate. It has the formula I:

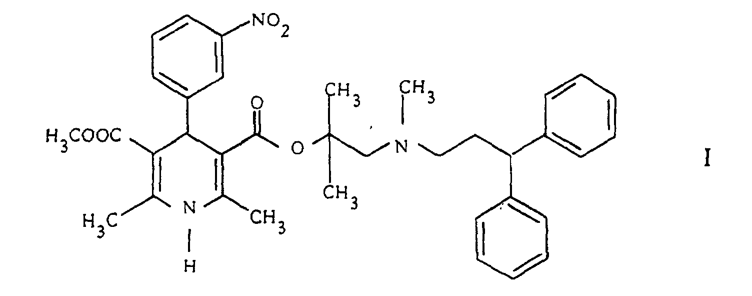

Lercanidipine was disclosed in United States Patent No 4705797. It is an antagonist of type-L calcium channels, and has been found to be very active as an antihypertensive and as an agent for the treatment of angina and coronary disease.

The preparation of lercanidipine, as described in the aforesaid US Patent, follows the route shown in the following reaction scheme:

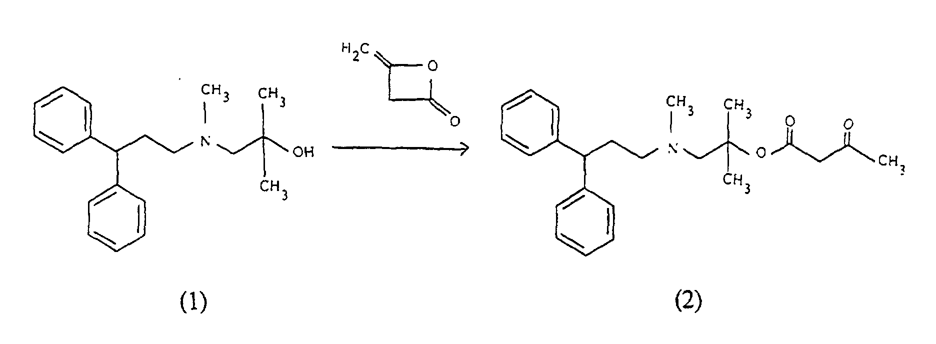

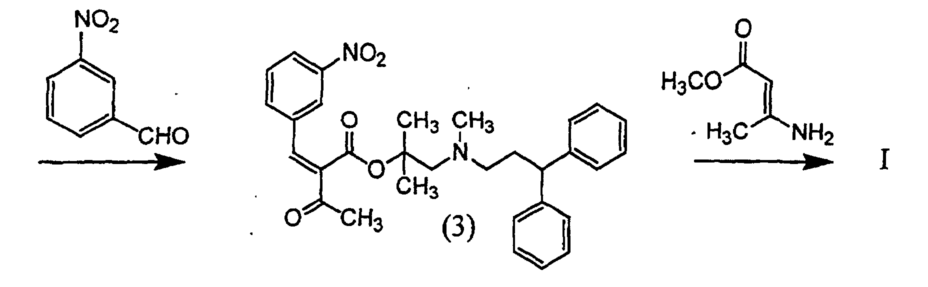

According to this scheme, the aminoalcohol (1) is esterified with diketene to form the corresponding acetoacetate (2), which is then coupled with 3-nitrobenzaldehyde to give 1,1,N-trimethyl-N-(3,3-diphenylpropyl)-2-aminoethyl α-acetyl-3-nitrocinnamate (3). This is cyclised with methyl 3-aminocrotonate in refluxing isopropanol.

This process has a number of disadvantages. By its very nature, the Hantzsch cyclisation used in the final step gives rise to several by-products. Not only does this reduce the yield of the desired product, but the removal of the by-products requires the use of purification techniques, such as column chromatography, which are difficult to apply on an industrial scale. Indeed, the yield for the final step is 35%, and the overall process yield is 23%.

The product obtained in US 4705797 by this process is lercanidipine hydrochloride hemihydrate, melting at 119 to 123°C. This product is somewhat hygroscopic, which can lead to inconstancy of composition and difficulties in handling during the preparation of pharmaceutical formulations. Moreover, the stability of the lercanidipine hydrochloride hemihydrate is not entirely satisfactory.

In Drugs of the Future, Vol. 12, No. 12, 1987, the same process was used, but the product was worked up in a different and undisclosed method (which in fact involved column chromatography and multiple crystallisations) to obtain anhydrous lercanidipine hydrochloride, melting at 186 to 188°C.

The invention provides a process for the preparation of lercanidipine hydrochloride, the process comprising:
a) halogenating 2,6-dimethyl-5-methoxycarbonyl-4-(3-nitrophenyl)-1,4-dihydropyridine-3-carboxylic acid with a halogenating agent in an aprotic solvent;
b) adding 2,N-dimethyl-N-(3,3-diphenylpropyl)-1-amino-2-propanol dissolved in an aprotic solvent to the resultant acid halide; and
c) isolating the resultant lercanidipine as its anhydrous hydrochloride.

The process of the invention is illustrated by the following reaction scheme.

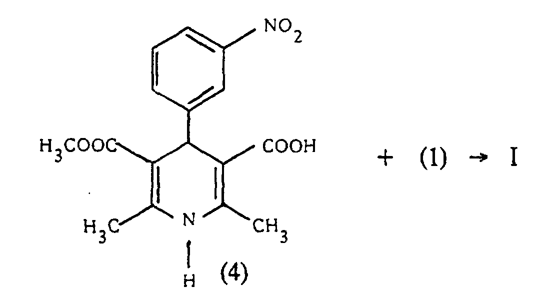

Preferably, the halogenation is a chlorination. It may be conducted in chlorinated or non-chlorinated aprotic solvents, e.g. chloroform, dichloromethane, dichloroethane, chlorobenzene, 1,1,1-trichloroethane, ethyl acetate, methyl acetate, tetrahydrofuran, dioxane, dimethylformamide, dimethyl carbonate or any mixture thereof, using known chlorinating agents, e.g. thionyl chloride, phosphorus pentachloride, phosphorus trichloride, phosphorus oxychloride, oxalyl chloride or other commercial chlorinating agents, operating at a temperature ranging from -15°C to +40°C, optionally in an inert gas atmosphere such as nitrogen or argon. The duration of the chlorination reaction may be from 15 minutes to 3 hours.

The aminoalcohol (1) dissolved in one of the above solvents or, alternatively, in another aprotic solvent, e.g. toluene, xylene or an alkane or cycloalkane having from 5 to 7 carbon atoms, is then added while keeping the reaction temperature in the -15°C to +40°C range and the reaction is allowed to continue until completed. Completion of the reaction may be determined by testing a sample from the reaction mixture by appropriate analytical techniques such as thin layer chromatography or high performance liquid chromatography.

The process of the invention is an esterification of the corresponding dihydropyridine acid. Since the acid halide does not need to be isolated, it is in effect a one-step process. Compared to the prior art process, described above, fewer reaction by-products are formed. As a result, an improved yield is obtained and this is accompanied by simpler purification and isolation of the lercanidipine. This may be conducted by conventional methods based, for instance, on extraction with solvents from basified solutions, re-salification with hydrochloric acid and re-crystallisation. Thus the use of chromatographic columns to isolate the desired final product can be avoided. As column chromatography always requires the use of high amounts of organic eluants, its avoidance clearly contributes to the industrial applicability of the process in terms of improved product quality, lower manufacturing costs and easier ecological disposal of process waste.

The lercanidipine hydrochloride prepared according to the present invention is in an anhydrous crystalline form and melts within two degrees Celsius in the 185 to 190°C range after re-crystallization of the crude hydrochloride first from aprotic solvents, such as ethyl acetate, methyl acetate or acetone, and then from protic solvents, such as one or more of methanol, ethanol, n-propanol, i-propanol, n-butanol, s-butanol and t-butanol, optionally in admixture with other solvents, including water. It has furthermore been found to be more stable and less hygroscopic than the lercanidipine hydrochloride hemihydrate obtained according to the prior process. These properties make it more suitable for pharmaceutical use, since they facilitate simpler large-scale manufacture of solid pharmaceutical preparations.

The following Examples illustrate the invention.

### EXAMPLE 1

### Lercanidipine hydrochloride

45.8 g (0.385 mol) of thionyl chloride were added dropwise over a period of about 15 minutes to a stirred mixture of 116.2 g (0.35 mol) of 2,6-dimethyl-5-methoxycarbonyl-4-(3-nitrophenyl)-1,4-dihydropyridine-3-carboxylic acid (4), prepared as described in German Patent 2847237, 645 ml of anhydrous dichloromethane and 160 ml of anhydrous dimethylformamide kept at a temperature of -4 to +1°C under nitrogen. The mixture was stirred for 1 hour within the same temperature range. A solution of 104.1 g (0.35 mol) of 2,N-dimethyl-N-(3,3-diphenylpropyl)-1-amino-2-propanol (1), prepared as described in US Patent 4705797, in 105 ml of anhydrous dichloromethane was then added dropwise over a period of about 15 minutes at -10 to 0°C. After stirring for 3 hours at 0°C and standing for 18 to 20 hours at ambient temperature, the solvent was evaporated off *in vacuo* and the residue was dissolved in 3500 ml of ethyl acetate. The organic solution was washed sequentially with a saturated NaCl solution (700 ml), 10% Na₂CO₃ (700 ml x 5), saturated NaCl solution (700 ml), 1N HCI (700 ml x 5) and saturated NaCl solution (700 ml). The organic layer was separated off, dried over anhydrous Na₂SO₄ for 30 minutes, filtered, treated with 23 g of carbon and re-filtered. The resulting solution was then concentrated in vacuo to a volume of about one litre and seeded with lercanidipine hydrochloride crystals. After standing for one day at 0 to 5°C, the solid was filtered and re-crystallized from absolute ethanol to give 179.5 g (78% of theory) of lercanidipine hydrochloride with a melting point of 186-188°C.

### EXAMPLE 2

### Stability at 100°C in light

Samples of anhydrous lercanidipine hydrochloride, prepared as described in Example 1, and lercanidipine hydrochloride hemihydrate, prepared as described in US Patent No 4705797, were heated at 100°C for 48 hours. The samples were checked at 0, 24 and 48 hours by HPLC analysis using the following conditions:

| | |
|---|---|
| Column | m-Bondapak C-18 (Waters), particle size 10 mm, 300 x 3.9 mm internal diameter. |
| Eluant | CH₃CN (61%) : 0.15 M NaClO₄ aqueous solution brought to pH 3 by adding HClO₄ (39%), (v/v) |
| Elution | isocratic |
| Flow | 1.5 ml/min |
| Temperature | 25°C |
| Detector | UV (240nm) |
| Attenuation | 0.05 AUFS |

Under these conditions, the retention time of lercanidipine HCl was about 7 minutes. The results are reported in Table I.

**TABLE I**

| | | HPLC purity (%) | | |
|---|---|---|---|---|
| | | Initial | 24 Hours | 48 hours |
| 100°C light | anhydrous | 99.74 | 99.36 | 99.01 |
| | hemihydrate | 99.85 | 92.35 | 90.96 |

It is clear that the anhydrous form of lercanidipine hydrochloride is remarkably more stable than the hemihydrate.

### EXAMPLE 3

### Stability at 40°C and at 60°C under 75% Relative Humidity in the dark

Samples of the two different forms of lercanidipine hydrochloride, as identified in Example 2, were placed in open polyethylene bags inserted in open glass flasks kept at 60°C under 75% relative humidity. The samples were checked for hygroscopicity, determining water content by the Karl Fisher (K.F.) method at 0, 8 and 15 days. The experiment was repeated at 40°C under 75 % relative humidity. The results are reported in Table II.

**TABLE II**

| | | Water Content (%) - K.F. | | |
|---|---|---|---|---|
| | | Initial | 8 Days | 15 Days |
| 60°C dark | anhydrous | 0.28 | 0.85 | 0.77 |
| | hemihydrate | 1.42 | 4.00 | 4.04 |
| 40°C dark | anhydrous | 0.28 | 0.30 | 0.32 |
| | hemihydrate | 1.42 | 3.14 | 3.05 |

The anhydrous form of lercanidipine hydrochloride is clearly less hygroscopic than the hemihydrate.

## Claims

1. A process for the preparation of methyl 1,1,N-trimethyl-N-(3,3-diphenylpropyl)-2-aminoethyl 1,4-dihydro-2,6-dimethyl-4-(3-nitrophenyl)-pyridine-3,5-dicarboxylate (lercanidipine) hydrochloride, the process comprising:
a) halogenating 2,6-dimethyl-5-methoxycarbonyl-4-(3-nitrophenyl)-1,4-dihydropyridine-3-carboxylic acid with a halogenating agent in an aprotic solvent;
b) adding 2,N-dimethyl-N-(3,3-diphenylpropyl)-1-amino-2-propanol dissolved in an aprotic solvent to the resultant acid halide; and
c) isolating the resultant lercanidipine as its anhydrous hydrochloride.

2. A process according to claim 1 wherein the halogenating agent is thionyl chloride, phosphorus pentachloride, phosphorus trichloride, phosphorus oxychloride or oxalyl chloride.

3. A process according to claim 1 or claim 2 wherein the aprotic solvent in which the halogenation is effected is chloroform, dichloromethane, dichloroethane, chlorobenzene, 1,1,1-trichloroethane, ethyl acetate, methyl acetate, tetrahydrofuran, dioxane, dimethylformamide, dimethylcarbonate or a mixture of two or more thereof.

4. A process according to any preceding claim wherein the aprotic solvent used to dissolve the 2,N-dimethyl-N-(3,3-diphenylpropyl)-1-amino-2-propanol is chloroform, dichloromethane, dichloroethane, chlorobenzene, 1,1,1-trichloro-ethane, ethyl acetate, methyl acetate, tetrahydrofuran, dioxane, dimethylformamide, dimethylcarbonate, toluene, xylene, an alkane having from 5 to 7 carbon atoms, a cycloalkane having from 5 to 7 carbon atoms or a mixture of two or more thereof.

5. A process according to any preceding claim wherein the lercanidipine is isolated by crystallization.

6. A process according to claim 5 wherein the crystallization is carried out in two successive steps, alternating aprotic and protic solvents.

7. A process according to claim 6 wherein the aprotic crystallization solvent is ethyl acetate, methyl acetate or acetone.

8. A process according to claim 6 or claim 7 wherein the protic crystallization solvent is one or more of methanol, ethanol, n-propanol, i-propanol, n-butanol, s-butanol and t-butanol, optionally in admixture with water.

## Patentansprüche

1. Verfahren zur Herstellung von Methyl-1,1,N-trimethyl-N-(3,3-diphenylpropyl)-2-aminoethyl-1,4-dihydro-2,6-dimethyl-4-(3-nitrophenyl)-pyridin-3,5-dicarboxylat(lercanidipin)hydrochlorid, umfassend:
a) Halogenieren einer 2,6-Dimethyl-5-methoxycarbonyl-4-(3-nitrophenyl)-1,4-dihydropyridin-3-carbonsäure mit einem Halogenierungsmittel in einem aprotischen Lösungsmittel;
b) Zugabe von in einem aprotischen Lösungsmittel gelöstem 2,N-Dimethyl-N-(3,3-diphenylpropyl)-1-amino-2-propanol zu dem resultierenden Säurehalogenid; und
c) Isolieren des resultierenden Lercanidipins in der Form seines wasserfreien Hydrochlorids.

2. Verfahren nach Anspruch 1, wobei das Halogenierungsmittel Thionylchlorid, Phosphorpentachlorid, Phosphortrichlorid, Phosphoroxychlorid oder Oxalylchlorid ist.

3. Verfahren nach Anspruch 1 oder Anspruch 2, wobei das aprotische Lösungsmittel, in dem die Halogenierung erfolgt, Chloroform, Dichlormethan, Dichlorethan, Chlorbenzol, 1,1,1-Trichlorethan, Ethylacetat, Methylacetat, Tetrahydrofuran, Dioxan, Dimethylformamid, Dimethylcarbonat oder ein Gemisch von zwei oder mehreren davon ist.

4. Verfahren nach einem vorhergehenden Anspruch, wobei das zum Lösen des 2,N-Dimethyl-N-(3,3-diphenylpropyl)-1-amino-2-propanols verwendete aprotische Lösungsmittel Chloroform, Dichlormethan, Dichlorethan, Chlorbenzol, 1,1,1-Trichlorethan, Ethylacetat, Methylacetat, Tetrahydrofuran, Dioxan, Dimethylformamid, Dimethylcarbonat, Toluol, Xylol, ein Alkan mit 5 bis 7 Kohlenstoffatomen, ein Cycloalkan mit 5 bis 7 Kohlenstoffatomen oder ein Gemisch von zwei oder mehreren davon ist.

5. Verfahren nach einem vorhergehenden Anspruch, wobei das Lercanidipin durch Kristallisation isoliert wird.

6. Verfahren nach Anspruch 5, wobei die Kristallisation in zwei aufeinanderfolgenden Schritten, abwechselnd mit aprotischen und protischen Lösungsmitteln, durchgeführt wird.

7. Verfahren nach Anspruch 6, wobei das aprotische Kristallisations-Lösungsmittel Ethylacetat, Methylacetat oder Aceton ist.

8. Verfahren nach Anspruch 6 oder Anspruch 7, wobei das protische Kristallisations-Lösungsmittel eines oder mehrere aus der Gruppe Methanol, Ethanol, n-Propanol, i-Propanol, n-Butanol, s-Butanol und t-Butanol, gegebenfalls in Mischung mit Wasser, ist.

## Revendications

1. Procédé pour la préparation de chlorhydrate de 1,4-dihydro-2,6-diméthyl-4-(3-nitrophényl)pyridine-3,5-dicarboxylate de méthyl 1,1,N-triméthyl-N-(3,3-diphénylpropyl)-2-aminoéthyle (lercanidipine), le procédé comprenant :
a) l'halogénation d'acide 2,6-diméthyl-5-méthoxycarbonyl-4-(3-nitrophényl)-1,4-dihydropyridine-3-carboxylique avec un agent halogénant dans un solvant aprotique ;
b) l'addition de 2,N-diméthyl-N-(3,3-diphénylpropyl)-1-amino-2-propanol dissous dans un solvant aprotique à l'halogénure d'acide résultant ; et
c) l'isolement de la lercanidipine résultante sous la forme de son chlorhydrate anhydre.

2. Procédé selon la revendication 1, dans lequel l'agent halogénant est le chlorure de thionyle, le pentachlorure de phosphore, le trichlorure de phosphore, l'oxychlorure de phosphore ou le chlorure d'oxalyle.

3. Procédé selon la revendication 1 ou la revendication 2, dans lequel le solvant aprotique dans lequel l'halogénation est effectuée est le chloroforme, le dichlorométhane, le dichloroéthane, le chlorobenzène, le 1,1,1-trichloroéthane, l'acétate d'éthyle, l'acétate de méthyle, le tétrahydrofuranne, le dioxane, le diméthylformamide, le carbonate de diméthyle ou un mélange de deux ou plusieurs de ceux-ci.

4. Procédé selon l'une quelconque des revendications précédentes, dans lequel le solvant aprotique utilisé pour dissoudre le 2,N-diméthyl-N-(3,3-diphénylpropyl)-1-amino-2-propanol est le chloroforme, le dichlorométhane, le dichloroéthane, le chlorobenzène, le 1,1,1-trichloroéthane, l'acétate d'éthyle, l'acétate de méthyle, le tétrahydrofuranne, le dioxane, le diméthylformamide, le carbonate de diméthyle, le toluène, le xylène, un alcane ayant de 5 à 7 atomes de carbone, un cycloalcane ayant de 5 à 7 atomes de carbone ou un mélange de deux ou plusieurs de ceux-ci.

5. Procédé selon l'une quelconque des revendications précédentes, dans lequel la lercanidipine est isolée par cristallisation.

6. Procédé selon la revendication 5, dans lequel la cristallisation est effectuée en deux étapes successives, en alternant des solvants aprotique et protique.

7. Procédé selon la revendication 6, dans lequel le solvant de cristallisation aprotique est l'acétate d'éthyle, l'acétate de méthyle ou l'acétone.

8. Procédé selon la revendication 6 ou la revendication 7, dans lequel le solvant de cristallisation protique est un ou plusieurs parmi le méthanol, l'éthanol, le n-propanol, le i-propanol, le n-butanol, le s-butanol et le t-butanol, éventuellement mélangé avec de l'eau.
